# EUROPEAN PATENT APPLICATION

(11) **EP 4 742 248 A1**
(43) Date of publication of application: **13.05.2026**
(21) Application number: 24211885.9
(22) Date of filing: 08.11.2024
(51) Int. Cl.: G16B 40/20, B82Y 30/00, H10K 85/20

(54) **OPTIMIZING OPTICAL NANO-BIOSENSORS MADE OF SINGLE-WALLED CARBON NANOTUBES WRAPPED IN SINGLE-STRANDED SEQUENCES OF NUCLEOTIDES FOR DETECTION OF MOLECULES SUCH AS GLUCOSE AND CANCER BIOMARKERS**

(71) Applicant: ECOLE POLYTECHNIQUE FEDERALE DE LAUSANNE (EPFL), 1015 Lausanne (CH)
(72) Inventor: Boghossian, Ardemis Anoush, 1096 Cully (CH); Rabbani, Yahya, 1007 Lausanne (CH); Bregy, Joey, 2822 Courroux (CH)
(74) Representative: E. Blum & Co. AG

(57) **Abstract**

The invention is notably directed to a computer-implemented method of optimizing an optical sensor (1) comprising a single-walled carbon nanotube (2), or SWCNT for short, which is wrapped in single-stranded sequence (3) of nucleotides (ssSN). The method revolves around two or more optimization cycles. Each optimization cycle includes clustering ssSNs of an input set of ssSNs to obtain clusters and selecting representative ssSNs of the clusters obtained. Next, one accesses measurements of optical responses of optical sensors to a target molecule, where the optical sensors include SWCNTs wrapped in actual ssSNs, which are synthetized in accordance with the representative ssSNs selected. After that, a top ssSN is identified among the representative ssSNs. The top ssSN is one that led to the highest optical response. Importantly, the top ssSN identified at each optimization cycle (but the last cycle) is mutated to obtain mutants and form a superset of sequences including the top ssSN and its mutants, prior to performing the next optimization cycle, using this superset as a new input set of ssSNs. This way, a local optimum is identified in the initial input space of the ssSNs. Additional optimization can be achieved through supervised learning and pattern recognition. The invention is further directed to related engineering methods (to synthetize ssSNs and wrap SWCNTs) and computer programs (to perform the optimizations).

## Description

### TECHNICAL FIELD

The invention relates in general to the field computer-implemented methods of optimizing optical sensors comprising single-walled carbon nanotubes wrapped in single-stranded sequences of nucleotides (e.g., DNA sequences), as well as related methods of engineering optical sensors, and related computer programs.

The goal is to identify at least one sequence of nucleotides that shows a measurable response to a target molecule, such as glucose or a cancer biomarker, it being noted that most sequences will typically not respond, especially when dealing with small target molecules. In particular, the invention concerns a method that determines a local optimum in a large space of input DNA sequences, by clustering the sequences, selecting representative sequences in each cluster, and accessing measurements of optical responses of nano-biosensors wrapped in the selected sequences. Next, a top sequence is identified and mutated, to form a superset that serves as an input set of sequences for a next optimization cycle.

### BACKGROUND

Monitoring blood glucose is vital for diabetes management. However, current methods are often inconvenient or imprecise. Nano-biosensors made of single-walled carbon nanotubes (SWCNTs) wrapped in single-stranded sequences of DNA (ssDNA) can potentially be used as optical sensors for monitoring blood glucose, due to their transparency, photostability, sensitivity, and selectivity. SWCNTs, which can be represented as graphene sheets wrapped into cylinders, demonstrate remarkable physical, mechanical, and chemical capabilities, providing an excellent platform for designing nano-biosensors. However, identifying an optimal DNA sequence for glucose responsiveness within the vast space of possible sequences presents a significant challenge.

The development of ssDNA-SWCNT sensors faces major challenges, particularly in finding an initial DNA sequence that effectively responds to a target analyte. Researchers lack knowledge about how specific DNA sequences affect sensor properties, making it necessary to screen large DNA libraries, which is time-consuming and inefficient. Techniques like random screening and SELEX can identify sequences with strong binding affinities but often fail to achieve the desired fluorescence response needed for sensor applications

Moreover, as the present inventors realized, other target molecules could potentially be sensed using SWCNTs wrapped in single-stranded sequences of nucleotides, beyond glucose molecules. However, a similar problem remains. That is, it is necessary to determine one or more sequences of nucleotides that measurably respond, optically, to a target molecule. Therefore, novel methods are needed to optimize and engineer optical nano-biosensors as described above.

The following document forms part of the background art: Lambert, B. P. J. G. Directed evolution of DNA-wrapped single-walled carbon nanotube complexes for optical sensing. PhD thesis (EPFL, Lausanne, 2021). 10.5075/epfl-thesis8406.

### SUMMARY

According to a first aspect, the invention is embodied as a computer-implemented method of optimizing an optical sensor comprising a single-walled carbon nanotube (SWCNT), which is wrapped in a single-stranded sequence of nucleotides (ssSN). The method comprises performing two or more optimization cycles.

Each optimization cycle includes the following steps:
- First, ssSNs of an input set of ssSNs are clustered to obtain clusters. The clustering step may possibly be performed so as to achieve an optimal number of clusters. Representative ssSNs are then selected in each of the clusters obtained.
- Next, optical response measurements are accessed, which are measurements of optical responses of optical sensors to a target molecule. The optical sensors at issue include SWCNTs wrapped in actual ssSNs, where the latter have been synthetized in accordance with the representative ssSNs selected. Interestingly, use can potentially be made of nano sensor arrays. For example, high throughput screening and measurements of different ssDNA sequences and different chiralities of SWCNT can be performed, as in embodiments described later.
- A top ssSN is subsequently identified among the representative ssSNs. The top ssSN is an ssSN that led to the highest optical response among all the optical responses for which measurements were accessed.

Importantly, however, a step of mutation is performed between two optimization cycles. That is, the method further comprises mutating the top ssSN identified at each optimization cycle (but the last cycle) to obtain mutants and form a superset of sequences including (or consisting of) the top ssSN and the mutants obtained. This mutation is performed prior to performing a next optimization cycle, which will use this superset as a new input set of ssSNs. Note, several top ssSNs could actually be identified during each cycle.

As noted in the background section, one challenge is the lack of understanding of how sequences of nucleotide (e.g., DNA sequences) affect the optical response of the SWCNT (e.g., SWCNT fluorescence). This, together with the vast variability in sequences of nucleotides, complicates the design of analyte-specific sensors. For example, a 30-nucleotide sequence of ssDNA results in 4³⁰ possible combinations. Now, as the present inventors concluded, specific sequences of nucleotides can form stable conformations with certain SWCNT chiralities, causing fluorescence shifts in response to different target analytes, hence the need to try and identify sequences that response well, optically.

The clustering scheme makes it possible to adequately partition and sample the large input sequence space. Various optical measurements can be contemplated. Preferred is to rely on photoluminescence measurements. The selected ssSNs form a subset of the representative sequences identified earlier, in the interest of fast convergence. This way, the clustering scheme ensures an adequate sampling, i.e., a diverse selection for the subsequent screenings, while limiting the actual number of screening tests (only one or a few representative sequences are selected per cluster). Meanwhile, the mutation step performed between two consecutive cycles allows the sequence space to be adequately explored, locally, around the top sequences identified, making it eventually possible to optimize the detection response locally, i.e., where it is, *a priori,* the most promising. The above scheme guarantees that at least a local optimum is found. In addition to the above optimization cycles, ML and pattern recognition techniques can be applied to refine the sequences and enhance their responsiveness and specificity, as in embodiments discussed below. Application can notably be made to detection of a small molecule, such as glucose (obtained, e.g., from a blood sample). In variants, the present method is applied to detect cancer biomarkers such as osteoprotegerin (OPG). In other variants, the method is used to detect nitric oxide (NO) for inflammation monitoring purposes.

In embodiments, the step of mutating the top ssSN, the top ssSN is mutated several times through changes in both base and length. In other words, several mutations are applied within a specific region (corresponding to the top ssSN) of the large input sequence space to try and identify ssSNs leading to enhanced optical responses of the wrapped SWCNTs to the target molecule. This makes it possible to adequately explore and optimize the detection response locally. For example, a suitable practical scheme is the following: the top ssSN can be mutated by introducing *n_{bsm}* base sequence mutations and adding *n_{ba}* bases at extremities of the top ssSN, where each of *n_{bsm}* and *n_{ba}* is equal to 1, 2, or 3.

A given clustering scheme results in a single set of clusters. However, a preferred strategy is to obtain several sets of parallel clusters, which makes it possible to adequately span the input sequence space, from different viewpoints. So, in embodiments, the ssSNs are clustered, at each optimization cycle, according to one or more distance functions based on *N_{d}* distinct sequence properties of the ssSNs. As a result, the clusters obtained at each optimization cycle include at least *N_{d}* parallel sets of clusters, where *N_{d} >_* 2, and preferably *N_{d} =* 3. More than *N_{d}* parallel sets of clusters could be obtained, should different distance functions be used for each sequence property considered. Distinct distance functions can be used for each sequence property considered. The representative ssSNs are selected in each cluster of the distinct sets of clusters obtained. Each of the representative ssSNs is preferably selected based on its distance to the centre of the respective cluster. This results in parallel sets of clusters. And this eventually ensures a better diversity, inasmuch as representative ssSNs are then selected in each cluster of the various sets.

The clustering schemes can be adequately chosen to result in a fairly small number of sets, reflecting fairly different conceptual approaches. In practice, a satisfactory trade-off is to choose two or each of the following approaches, as in embodiments. Namely, the distinct sequence properties may include two or more, or preferably each, of the following properties: (i) an alignment similarity of the sequences, (ii) a k-mer frequency in the sequences, where k is preferably of between 3 and 7, and more preferably equal to 3 (notably when the target molecule is a small molecule such as glucose), and (iii) folding properties of the sequences.

In embodiments, each optimization cycle further comprises determining optimal numbers of clusters for respective ones of the *N_{d}* parallel sets, preferably using the Elbow method. The ssSNs are clustered in accordance with the optimal numbers of clusters determined. Optimizing the number of clusters makes it possible to optimize the number of experiments required in the real world. The Elbow method was found to be particularly simple and computationally efficient. In addition, it is believed to decrease the number of subsequent experiments, for reasons that are rather unintuitive, even if the Elbow method typically results in a larger number of clusters, initially. The clusters are preferably obtained through machine learning (ML) techniques, much preferably using an unsupervised ML clustering method, in the interest of speed. Use can for instance be made of the so-called x-means clustering method.

In embodiments, the method further comprises, after the last optimization cycle, determining one or more candidate ssSNs that potentially provide highest optical responses to the target molecule. Such candidate ssSNs are determined from at least a subset, preferably a full set of, all representative ssSNs corresponding to all optical sensors for which measurements of optical responses were accessed so far, throughout the optimization cycles. The candidate ssSNs are determined using inference techniques relying on one or more computational models. Such computational models can be run on a very large number of sequences. This way, newer sequences may be discovered, which potentially leads to better optical performance. The inference techniques typically rely on ML and pattern recognition.

In embodiments, said one or more computational models involve one or more ML models. The one or more candidate ssSNs are determined by training each model and then running each model for inference purposes. That is, each ML model is trained on a training dataset consisting of pairs of inputs and outputs. These inputs correspond to at least a subset of said all representative ssSNs, respectively, while the outputs are based on measurements of optical responses as accessed for respective ones of the optical sensors. Once trained, each ML model is run on a test set of ssSNs to infer respective optical response performances, whereby the one or more candidate ssSNs are eventually determined based on the optical response performances inferred.

In particularly efficient embodiments, the set of one or more ML models comprises an odd number of ML models (at least three ML models are involved), each configured as a classifier. The optical response performances are inferred as classifications into classes. The classes include, preferably consist of, a first class corresponding to a high optical response and a second class corresponding to a low optical response. Moreover, the method further comprises performing a majority vote based on the classifications obtained from each model of the set of ML models. This makes it possible to consolidate the classifications obtained. In particular, false positives can potentially be discarded, whereby a safer classification of the optical responses can eventually be obtained.

In embodiments, the method further comprises performing pattern recognition on test sequences of the test set of ssSNs based on the optical response performances inferred for the test set of ssSNs. The goal is to identify sequence patterns that respond best, optically, to the target molecule. The one or more candidate ssSNs are eventually determined based on the sequence patterns identified. Moreover, the method preferably comprises predicting one or more new sequences based on the identified patterns, whereby the one or more candidate ssSNs are eventually determined based on the one or more new sequences predicted. The sequence patterns preferably include patterns in terms of one or more, preferably two, and more preferably three, of k-mers, base positions, and lengths, of the test sequences.

According to another aspect, the invention is embodied as a method of engineering one or more optical sensors, each comprising an SWCNT wrapped in an ssSN. This engineering method can be regarded as a complementary method of the optimization method described above. Like before, the engineering method comprises performing two or more optimization cycles, which are counterpart of the optimization cycles described above. Each cycle now includes the four following steps. First, representative ssSNs, as selected during a corresponding optimization cycle of the optimization method, are received from a computerized system, which may for instance the computer system used to implement the first aspect of the invention. Second, actual ssSNs are wrapped around SWCNTs to obtain optical sensors, where the actual ssSNs are sequences that are (or have been) synthetized in accordance with the representative ssSNs selected. Third, optical responses of the optical sensors to a target molecule are characterized to obtain measurements of the optical responses. Again, use can be made of an array of nano sensors (i.e., an array of wrapped SWCNTs). Fourth, the measurements of the optical responses are forwarded to the computerized system for it to identify one or more top ssSNs (among the representative ssSNs) that led to highest optical responses of the optical sensors.

The actual ssSNs are preferably wrapped around the SWCNTs using an exchange protocol, allowing for high-throughput preparation of ssSN-SWCNT complexes and facilitating the preparation of low-volume samples, thus reducing the cost of various screenings.

In embodiments, these optical responses are characterized through near infrared (nIR) fluorescence spectroscopy. The optical responses include, for each of the optical sensors, a fluorescence peak shift and/or a fluorescence peak intensity change. In particular, such optical responses can be characterized for different chiralities of the SWCNTs and different ssDNA sequences as synthetized in accordance with the representative ssSNs selected. Each of the fluorescence peak shift and the fluorescence peak intensity change can be obtained for one or more predetermined excitation wavelengths. Various approaches can be contemplated. In simple cases, only the fluorescence peak intensity change is considered.

In embodiments, the target molecule is glucose (e.g., blood glucose, initially obtained from a human or animal blood sample) and the ssSNs are single-stranded sequences of DNA. In variants, the target molecule is a cancer biomarker, such as osteoprotegerin (OPG). As also noted earlier, the target molecule can be nitric oxide (NO); the purpose is then to monitor inflammation. According to a final aspect, the invention is embodied as a computer program comprising software code adapted to perform a method of optimizing an optical sensor according as described above when executed by processing means. The computer program is preferably embodied as a computer program code product.

### BRIEF DESCRIPTION OF THE DRAWINGS

These and other objects, features and advantages of the present invention will become apparent from the following detailed description of illustrative embodiments thereof, which is to be read in connection with the accompanying drawings. The illustrations are for clarity in facilitating one skilled in the art in understanding the invention in conjunction with the detailed description. In the drawings:
FIG. 1 schematically represents a general-purpose computerized system, suited for implementing one or more optimization steps as involved in embodiments of the invention;
FIG. 2 is a flowchart illustrating high-level steps of a method of optimizing an optical nano-biosensor comprising single-walled carbon nanotubes (SWCNTs) wrapped in single-stranded sequences of nucleotides (ssSNs), as in embodiments;
FIG. 3A is a schematic view of an optical nano-biosensor comprising a SWCNT wrapped in a single-stranded DNA (ssDNA) sequence, receiving excitation light and emitting nIR emission light, as involved in embodiments;
FIG. 3B is a graph showing the relationship between intensity and wavelength, highlighting both an intensity change and a peak shift, as exploited to obtain optical responses of optical nano-biosensors in embodiments;
FIG. 4A schematically illustrates a feature extraction process, whereby ssDNA sequences are encoded as vectors, corresponding to points in a multidimensional space (here a 2D space is considered, for convenience), as involved in embodiments;
FIG. 4B schematically illustrates how ssDNA sequences are clustered, using three different clustering approaches, which results in three parallel sets of clusters, as in embodiments;
FIG. 4C schematically illustrates how a given ssDNA sequence can be mutated in base and length, as involved in embodiments;
FIG. 5A illustrates a machine learning (ML) process of response classification that involves a feature extraction from given DNA embeddings of test DNA sequences, and a subsequent training of a deep neural network, with a view to running the trained network and classify optical responses of several test sequences as "high" or "low", as involved in embodiments to further optimize ssDNA sequences for nano-biosensors;
FIG. 5B schematically illustrates a process of screening DNA-wrapped nanotubes, as involved in embodiments;
FIG. 5C is a graph illustrating optical responses of optical sensors in the multidimensional space (a 2D space) of FIG. 4A, as predicted using ML and pattern recognition techniques, as in embodiments;
FIG. 6A schematically illustrates three different sequence spaces (corresponding to distinct sequence properties) used to implement three distinct clustering schemes, respectively, where the sequence properties correspond to: (i) a k-mer frequency in the sequences, (ii) folding properties of the sequences, and (iii) an alignment similarity of the sequences, the latter obtained through the so-called MAFFT (multiple alignment using fast Fourier transform) method, as in embodiments;
FIG. 6B is graph showing the normalized WCSS (within-cluster sum of squares) as a function of the number of clusters for the different sequence properties noted above, where the resulting curves are used to implement the so-called elbow method, as in embodiments;
FIG. 6C is a graph illustrating experimental (9, 4) intensity changes in response to glucose detection at 7.5 mM, using SWCNTs wrapped in diverse ssDNA sequences as selected throughout an optimization based on a clustering scheme, according to embodiments; and
FIG. 7 schematically represents a system, suited for implementing one or more optimization and engineering cycles, as in embodiments. Each cycle involves a computerized system, an optical device to collect measurements of optical responses, and a laboratory to synthetize ssDNA sequences and wrap SWCNTs.

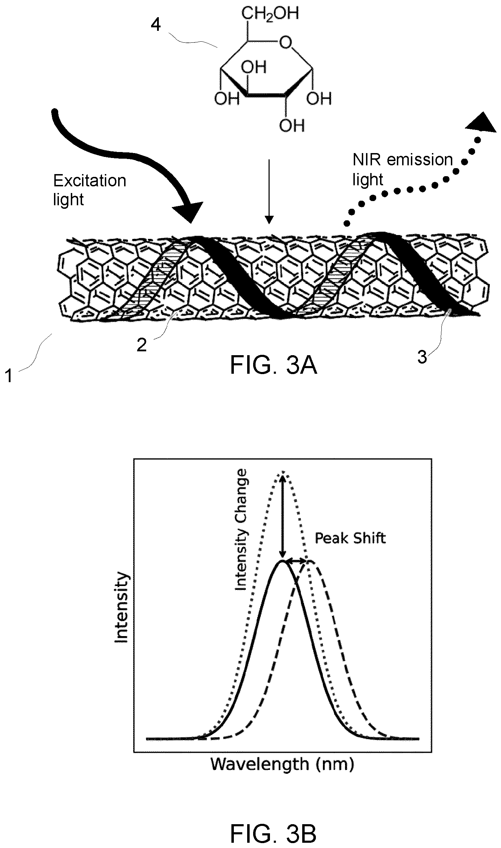

The accompanying drawings show simplified representations of devices or parts thereof, as involved in embodiments. FIG. 3A is not necessarily to scale. Similar or functionally similar elements in the figures have been allocated the same numeral references, unless otherwise indicated.

Methods and computer programs embodying the present invention will now be described, by way of non-limiting examples.

### DETAILED DESCRIPTION OF EMBODIMENTS OF THE INVENTION

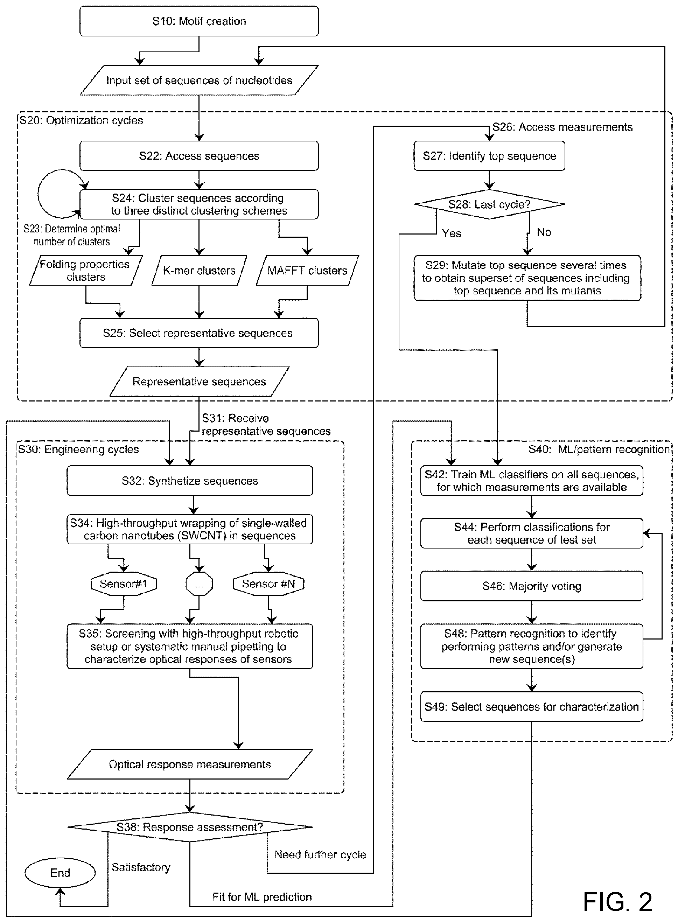

The following description is structured as follows. General embodiments and high-level variants are described in section 1. Section 2 addresses particularly preferred embodiments. Section 3 concerns technical implementation details. Note, the present method and its variants are sometimes collectively referred to as the "present methods". All references Sn refer to methods steps of the flowcharts of FIG. 2, while numeral references pertain to devices, components, and concepts involved in embodiments of the present invention.

### 1. General embodiments and high-level variants

A first aspect of the invention is now described in reference to FIG. 2. This aspect concerns a computer-implemented method of optimizing an optical sensor 1. An example of such an optical sensor 1 is shown in FIG. 3A. This sensor 1 includes a single-walled carbon nanotube (SWCNT) 2, which is wrapped in single-stranded sequence of nucleotides (ssSN) 3. In this example, the sensor 1 is meant to detect glucose 4, i.e., a target molecule, which may be present in a blood sample obtained from a human or animal.

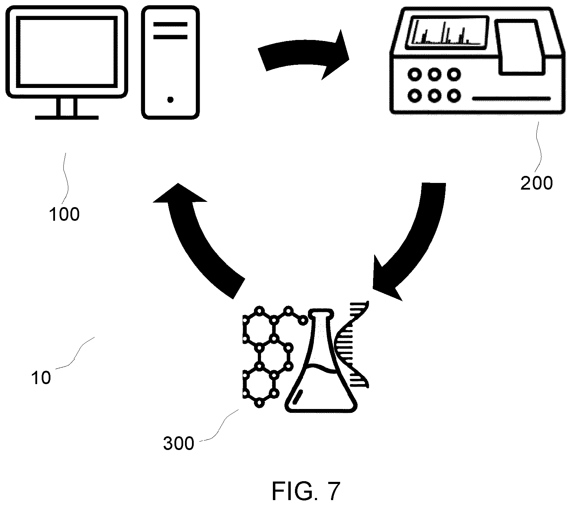

The proposed method revolves around performing two or more optimization cycles, as illustrated in FIG. 7. As shown in the detailed flow of FIG. 2, each optimization cycle (denoted by S20 in FIG. 2) includes the following steps, which are based on a given input set of ssSNs. This input set can be obtained by generating a (large) number of sequences, using any suitable sequence generation method. For example, two million sequences with different lengths and base compositions may initially be generated by using a motif creation technique or by randomly generating ssSNs. If necessary, constraints may be applied, e.g., in terms if GC content, Shannon entropy, and/or Kolmogorov-Chaitin complexity.

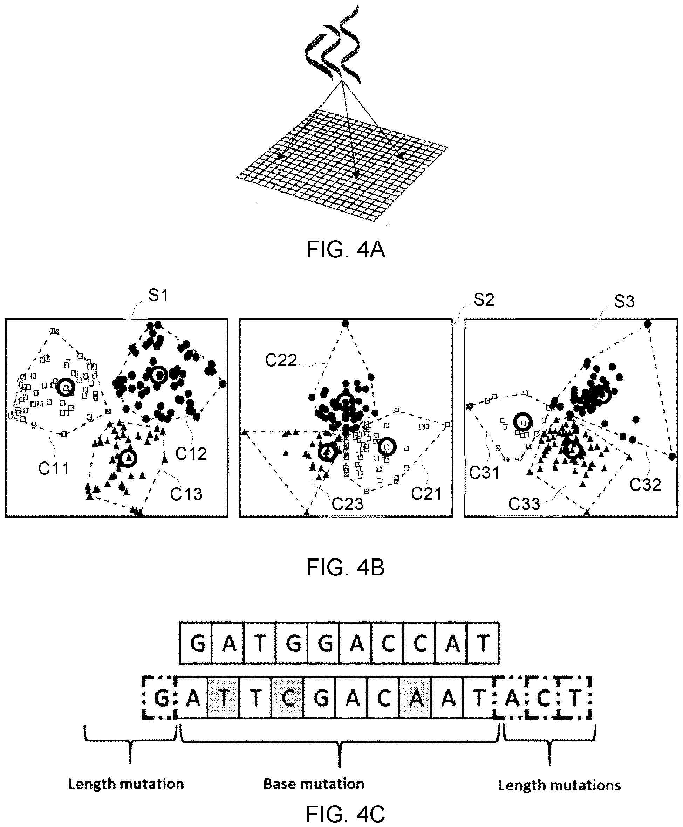

First, ssSNs of the input set are clustered (step S24) to obtain clusters. The clusters obtained are denoted by references C11 - C33 in FIG. 4B, where several clustering schemes are considered. Each data point corresponds to a vector (or array) extracted from a respective input sequence and projected on a 2D space map in this example, for the sake of illustration. In addition, representative ssSNs of the clusters obtained are selected at step S25. That is, for each of the cluster obtained, at least one representative ssSN is selected. The representative ssSNs selected are denoted by a black circle in FIG. 4B. Only one representative ssSN is selected for each cluster in this example. More generally, though, one or more representative sequences may be selected for each cluster C11 - C33 at step S25. Preferably, the selected ssSN are the most central sequences in each cluster. Alternatively, the ssSNs can be randomly selected. Other selection schemes can be contemplated, which strive to identify a most representative ssSN of each cluster (e.g., based on a certain k-mer frequency). This clustering scheme makes it possible to adequately partition and sample the large input sequence space.

Measurements of optical responses of optical sensors 1 are then accessed at step S26. The optical responses are responses of sensors 1 to a given target molecule 4 (e.g., glucose in FIG. 3A). The optical sensors 1 at issue include SWCNTs that have been wrapped in actual (i.e., physical) ssSNs, where the latter are assumed to have been synthetized in accordance with the representative ssSNs as selected at step S25. This method therefore involves carrying out experimental work between steps 25 and 26. Alternatively, the method could exploit previous experiments, the results of which could for example be available in a database.

The next step S27 aims at identifying a top ssSN among the representative ssSNs, i.e., an ssSN that led to the highest optical response. The top ssSN is identified according to one or more predetermined experimental criteria, which depend on the characterization technique used. This is discussed later in detail. Note, at least one top ssSN is identified among the representative ssSNs at step S27. That is, two or more top ssSNs could possibly be identified and selected at this step. Thus, several top ssSNs may possibly be selected, at each cycle, something that may also depend on the experimental criteria adopted. However, the selected ssSNs will always be a subset of the representative sequences identified earlier, in the interest of fast convergence.

Interestingly, the top ssSN(s) identified at step S27 are then mutated, step S29. The mutation step is illustrated in FIG. 4C. The goal is to obtain several mutants. A superset of sequences is then gathered, which include the top ssSN(s) identified at step S27 and the mutants obtained at step S29. A next optimization cycle can then be performed S20, using the superset obtained as a new input set of ssSNs. Note, the mutation step S29 need not be performed after the last optimization cycle. Preferably, exactly two optimization cycles are performed, which turns out to be sufficient in practice.

In addition to the above optimization cycles, ML and pattern recognition techniques can be applied to refine the sequences and enhance their responsiveness and specificity, as in embodiments discussed below. For example, use can be made of artificial neural networks (ANNs) and, in particular, convolutional neural networks (CNNs), graph neural network (GNNs). and graph convolutional network (GCNs).

The steps of accessing, clustering, and mutating sequences are performed through computerized means. So does the step of identifying the top ssSNs. Such steps make use of digital representations of the ssSNs. However, the above method also implies that complementary steps are performed in the physical world, to wrap actual sequences around nanotubes and characterize optical responses of the resulting sensors, using a suitable set-up, as illustrated in FIG. 7. The actual sequences refer to physical sequences of nucleotides, i.e., sequences in the real world. Such sequences are synthetized according to their abstract (or digital) counterparts, i.e., the representative sequences as selected at step S25.

Various optical measurements can be contemplated. Preferred is to rely on photoluminescence measurements and, in particular, fluorescence measurements. However, depending on the target molecules, other types of luminescence could be relied on, such as electroluminescence and chemiluminescence, e.g., electrochemiluminescence. More generally, other spectroscopy techniques could be employed. Steps performed in the "real world" concern another aspect of the invention, which is discussed below in detail.

Application can notably be made to detection of glucose (e.g., in blood samples). In variants, the present method is applied to detect nitric oxide (NO) and osteoprotegerin (OPG) cancer biomarkers. In such cases, the ssSNs are preferably DNA sequences. The DNA sequences selected can for instance be wrapped on the surfaces of the SWCNTs through a high-throughput exchange method. Moreover, photoluminescence properties of the ssDNA-SWCNT are preferably examined at two different excitation wavelengths, with a view to, e.g., capturing different photoluminescence properties of different chiralities of the nanotubes by high-throughput screening of different molecules such as glucose, NO or OPG cancer biomarkers. Various carbon nanotube chiralities can be considered, e.g., any subset of, or each of, the (6, 5), (7, 5), (7, 6), (10, 2), and (9, 4) chiralities. In variants to DNA, use can be made of RNA or XNA, for example.

Applications to NO sensing preferably make use of CNN models, in addition to clustering and pattern recognition techniques, to refine the sequences, while applications to OPG cancer biomarker sensing preferably make use of both ANNs (in particular CNN models), in addition to clustering, as discussed later in detail.

The clustering scheme ensures a diverse selection for the subsequent screenings, while limiting the actual number of screening tests. In detail, the clustering scheme approach allows an efficient screening of the entire input sequence space, which drastically reduces the number of experiments required in the physical world, to the extent that only one or a few representative sequences are selected per cluster. Nevertheless, the selecting of representative sequences in each cluster makes it possible to maintain a diverse sequence selection for the subsequent screening steps. Meanwhile, the mutation step performed between two consecutive cycles allows the sequence space to be adequately explored, locally, around the top sequences identified, making it eventually possible to optimize the detection response locally, i.e., where it is, *a priori,* the most promising.

While the above scheme cannot guarantee that an absolute optimum is found, it ensures that at least a local optimum is found. Moreover, supervised machine learning and pattern recognition techniques can be complementarily applied to all sequences for which measurements were obtained (or a subset thereof) to test a very large number of sequences in silico and identify further sequences with enhanced detection responses.

All this is now described in detail, in reference to particular embodiments of the invention. To start with, the top ssSN is preferably mutated S29 several times after each optimization cycle but the last one, see FIG. 2. Such mutations are achieved through changes in both base and length, as illustrated in FIG. 4C. In other words, mutations are applied within a specific region (corresponding to the top ssSNs) of the large input sequence space to try and identify ssSNs leading to enhanced optical responses of the wrapped SWCNTs to the target molecule. For example, up to three mutations in the bases and up to three mutations in the lengths at the 3' or 5' ends of single-stranded DNA. In variants, more than three mutations in length can be contemplated on both sides, something that can be advantageous to detect different analytes.

As further illustrated in FIG. 4C, the top ssSN is preferably mutated S29 by introducing *n_{bsm}* base sequence mutations and adding *n_{b},* bases at extremities of the top ssSN. In principle, each of *n_{bsm}* and *n_{ba}* may be equal to 1, 2, or 3. For example, application can be made to optimize glucose-responsive DNA sequences by applying mutations within a specific region of a large input DNA space, to enhance the response of ssDNA-SWCNT to glucose. A library of mutants can for instance be generated by introducing base sequence mutations (1 to 3 mutations) and adding bases at the extremities (1 to 3 bases). In the example of FIG. 4C, three bases are added at the extremities (GAG and ACT), while base sequence mutations are introduced at other (undefined) locations.

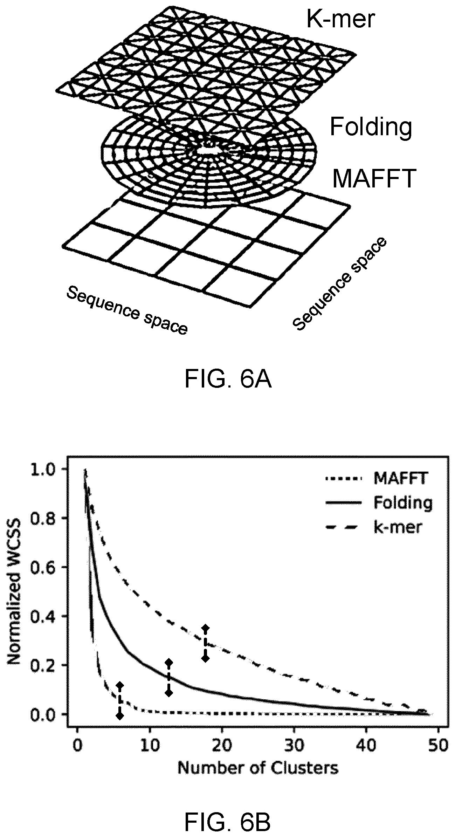

A given clustering scheme results in a single set of clusters. However, a preferred strategy is to obtain several sets S1 - S3 of clusters C11 - C33, as discussed below in detail. For example, referring to FIGS. 4B and 6A, the ssSNs are advantageously clustered S24 according to one or more distance functions (or, equivalently, one or more similarity measures) based *on N_{d}* distinct sequence properties of the ssSNs. That is, any selected sequence property gives rise to a respective vector, corresponding to a point in a multidimensional space. Then, a given distance function can be used to compute distances between such vectors and thereby allow a respective set of clusters to be computed. As a result, the clusters obtained at each optimization cycle include at least *N_{d}* parallel sets S1 - S3 of clusters, where *N_{d}* >_ 2 and, preferably, *N_{d} =* 3. Interestingly, the representative ssSNs are selected S25 in each cluster of the distinct sets of clusters obtained. Note, each representative ssSN is preferably selected S25 based on its distance to the centre of the respective cluster. For example, in FIG. 4B, one representative ssSNs (the most central sequence) is selected in each cluster C11 - C33 of the distinct sets S1 - S3.

Various sequence properties can be used to obtain the clusters. In embodiments, the distinct sequence properties include: (i) an alignment similarity of the sequences, (ii) a k-mer frequency in the sequences, where k is preferably of between 3 and 7, and more preferably equal to 3 (especially for small molecule), and (iii) folding properties of the sequences. In particular, two or three of the above properties may be used to form the clusters. The alignment similarity may for instance be obtained through the so-called MAFFT (multiple alignment using fast Fourier transform) method. Frequencies of k-mers are routinely computed in bioinformatics. Folding properties may for instance be extracted thanks to methods of predictions and analyses of secondary structures, such as the so-called ViennaRNA Package.

In the above examples, the *N_{d}* distinct properties correspond to physical properties identified from the sequences. However, in variants, the *N_{d}* distinct properties may correspond to, or include, features extracted (in a machine-learning sense) from the sequences, e.g., using any suitable feature extractor, which may possibly have been purposely trained on ssSNs. A same distance function can be used for each of the *N_{d}* distinct properties (or distinct sets of features). Alternatively, distinct distance functions (or, equivalently, distinct similarity measures) could be used for the *N_{d}* distinct properties. The distance functions can be selected in accordance with the properties or features considered. Another option is to combine distinct distance functions (or, equivalently, the similarity measures) with distinct sequence properties (or distinct sets of features). For example, if two distance functions are used for each of the *N_{d}* distinct properties, then the clusters obtained at each optimization cycle include 2 *N_{d}* parallel sets of clusters.

As one understands, several sets S1 - S3 of clusters C11 - C33 can be obtained in parallel, using one or more distance functions, as well as distinct physical properties (and/or distinct sets of features). As a result, the sets S1 - S3 of clusters C11 - C33 overlap in the sequence space, to the extent that each of the *N_{d}* sets S1 - S3 of clusters spans the entire input set of sequences. Using parallel sets of clusters eventually ensures a better diversity, inasmuch as representative ssSNs are then selected in each cluster of the various sets S1 - S3.

Still, the same clustering method may eventually be used. This clustering method is preferably an unsupervised clustering method, e.g., such as the so-called k means method. The latter aims to partition *N* observations into *k* clusters C11- C33, in which each observation belongs to the cluster with the nearest mean. The sequence that is the closest to the mean of each cluster may then be selected as a representative sequence, as in preferred embodiments. Note, the k means clustering method is hereafter referred to as the *κ*-means, using the Greek letter *κ*, to distinguish *κ* from the length k of substrings i.e., corresponding to the k-mers contained within a sequence.

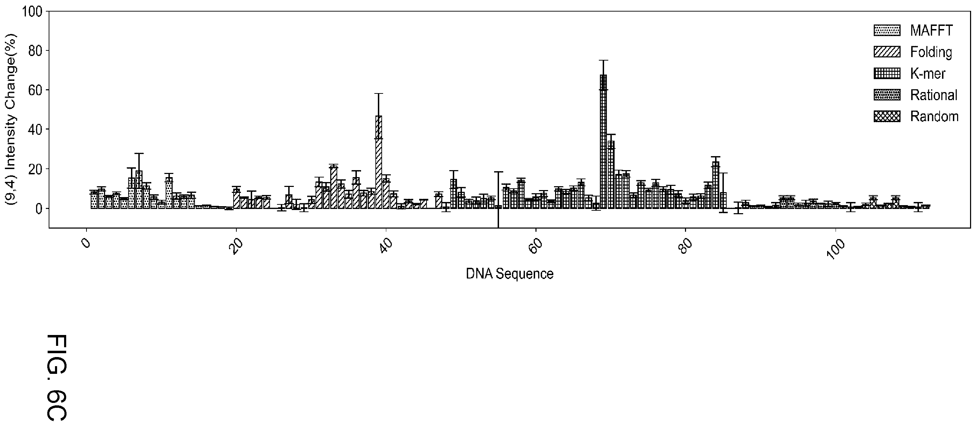

Referring now to FIG. 6C, each optimization cycle may further comprise a step S23 of determining optimal numbers of clusters for respective sets of the *N_{d}* parallel sets S1 - S3. This is preferably achieved using the so-called Elbow method. Accordingly, the ssSNs are clustered S24 in accordance with the optimal numbers of clusters determined, preferably using an unsupervised machine learning clustering technique, such as the *κ*-means clustering method. In variants to the Elbow method, other heuristics may be used, e.g., based on the silhouette score, gap statistics, or a dendrogram analysis. However, the elbow method was found to be particularly simple and computationally efficient. In addition, it is believed to decrease the number of subsequent experiments. The reason is rather unintuitive. Indeed, method based on silhouette scores and gap statistics tend to result in fewer clusters, compared with other methods. As a result, such method would *a priori* be preferred, be it to minimize the number of subsequent optical response measurements. In comparison, the Elbow method typically results in a larger number of clusters, initially. However, this also results in a finer partitioning, i.e., a finer sampling of the sequence space. In turn, this improves the chance of identifying a high-response sequence and subsequently requires less mutations to identify a local maximum. All in all, the Elbow method is believed to results in faster convergence.

Referring now to FIG. 2, embodiments of the method further involve, after the last optimization cycle, a step S40 of determining one or more candidate ssSNs that potentially provide highest optical responses to the target molecule. In the following, it is assumed that several candidate ssSNs are to be determined, for the sake of illustration. Such candidate ssSNs are determined from at least a subset, or preferably the full set of, the tested ssSNs. The tested ssSNs refer to those representative ssSNs corresponding to the optical sensors 1 for which measurements of optical responses were accessed throughout the optimization cycles. The candidate ssSNs are determined using inference techniques (steps S44, S48), which rely on one or more computational models. Different strategies may be contemplated. One possibility is to use empirical models or *ab initio* calculations, for example. A preferred option, however, is to rely on a data-driven approach. In particular, machine learning (ML) may again be used, albeit in a supervised context, as discussed below. Eventually, the candidate ssSNs determined may then be used to wrap actual ssSNs (synthetized in accordance with the candidate ssSNs determined) around SWCNTs, to obtain optimized optical sensors 1.

The models used at steps S44 and S48 preferably involve one or more ML computational models. The candidate ssSNs can be determined S40 by training S42 each ML model on a training dataset. As usual, the training dataset may consist of pairs of inputs and outputs. In the present context, the inputs considered will correspond to respective ssSNs, corresponding, or selected from, all the representative ssSNs identified so far. The associated outputs are based on measurements of the optical responses of the corresponding optical sensors 1, i.e., the measurements accessed during the various steps S26. Once trained, each ML model can be run S44 on a test set of ssSNs to infer S44 respective optical response performances. Eventually, the candidate ssSNs are determined S48 based on the optical response performances inferred.

In principle, such inferences can be predictions, i.e., in the form of a number (or a set of numbers) reflecting predicted optical responses. The inferences performed may alternatively be classifications, e.g., as high or low optical responses, meaning satisfactory or unsatisfactory responses. This classification is not necessarily binary, although it preferably is, as in embodiments discussed below in detail. Note, the test set can be identical to the input set of sequences as initially accessed at step S22 (see FIG. 2). Alternatively, the test set can be a subset or a superset of the initial set, or even a distinct set. Advantageously, the trained ML models can be run on a very large number of sequences. This way, newer sequences may be discovered, which potentially leads to better optical performance.

Various ML approaches can be contemplated to train and obtain a satisfactory model capable of predicting or classifying sensor responses for different sequences. The best performance was obtained from neural networks (NNs). The present inventors have tested several approaches, e.g., based on CNNs, GNNs, and GCNs. The resulting models were then used to predict the behaviour of new sequences, improve their responses, and identify further sequences offering improved optical response performance. In simpler variants, several types of networks are tested and trained for classification purposes, followed by a majority voting, as discussed below.

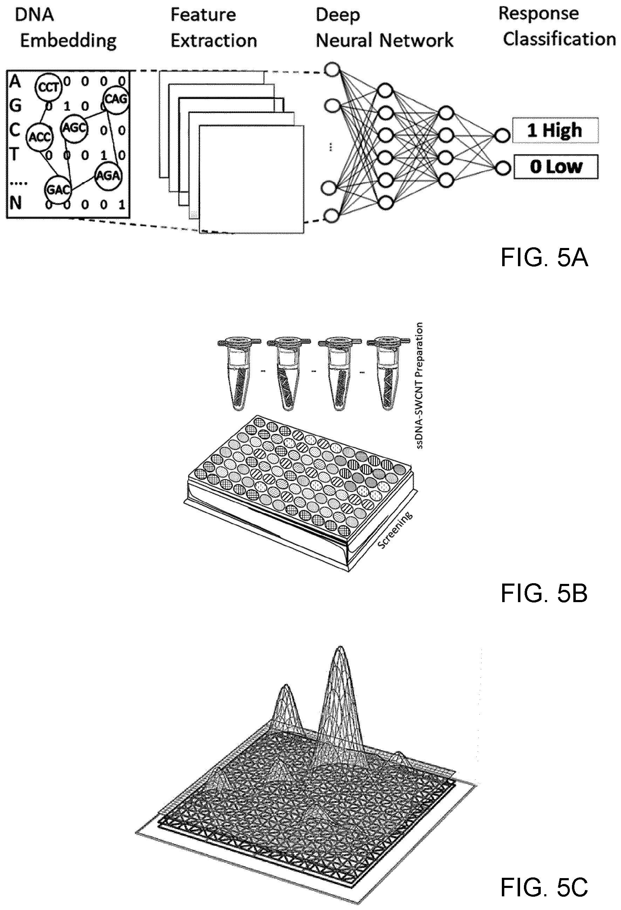

In embodiments, the set of one or more ML models comprises an odd number (at least three) of ML models, where each model is configured as a classifier. In that case, the optical response performances of the sequences are inferred S44 as classifications into classes. As noted above, the output classes preferably consist of two classes (binary classification). The first class corresponds to a high (i.e., satisfactory) optical response ("1 High" in FIG. 5A), while the second class corresponds to a low (i.e., insufficient) optical response ("0 Low" in FIG. 5A). Next, a majority vote is performed S46 based on the classifications obtained from each classifier. This makes it possible to consolidate the classifications obtained. In particular, false positives can potentially be discarded, whereby a safer classification of the optical responses can eventually be obtained.

Interestingly, the above steps S44, S46 of classification and majority voting can be followed by further steps, relying notably on pattern recognition. Namely, in embodiments, the present methods further comprise performing S48 pattern recognition on test sequences of the test set of ssSNs based on the optical response performances inferred for such sequences. The goal is to identify sequence patterns that respond best, optically, to the target molecule. Eventually, the candidate ssSNs are determined based on the sequence patterns identified.

To achieve this, one may advantageously try to predict new sequences based on the identified patterns. For example, pattern recognition can be applied to identify patterns including k-mers, as well as base and length positions within the sequence that respond best to the target molecule (e.g., glucose). In that case, the candidate ssSNs are eventually determined based on the new sequences predicted.

For example, pattern recognition can be used to generate all possible new DNA sequences that may have a high response to glucose. The response of these sequences can then be predicted using ML to identify a few promising sequences for experimental validation, if necessary. Thus, ML steps and pattern recognition steps can be intertwined, as suggested by the loop S48 to S44 in FIG. 2.

For instance, a neural network can first be employed for predicting new DNA sequences, while pattern recognition can be subsequently performed to identify S48 the best k-mers and analyse the positions of DNA spots that best interact with glucose. For example, such sequence patterns may include patterns in terms of k-mers, base positions, and/or lengths, of the test sequences. The new sequence identified S48 can then be fed S44 to a ML classifier, and so on.

Referring to FIG. 2, a complementary aspect of the invention concerns a method of engineering one or more optical sensors 1. Consistently with the first aspect of the invention, each sensor 1 comprises an SWCNT 2 wrapped in an ssSN 3. As before, the method comprises optimization cycles. However, each cycle now comprises complementary steps, i.e., steps carried out in the physical world. Namely, each cycle first comprises receiving S31 representative ssSNs from a computerized system, e.g., the very computerized system 100 used to perform steps according to the first aspect, or a system in data communication with that computerized system. The representative ssSNs are those ssSNs that are selected during a corresponding optimization cycle of the method according to the first aspect of the invention.

Next, actual ssSNs are wrapped S34 around SWCNTs, with a view to obtaining actual optical sensors. The actual ssSNs are sequences that are synthetized S32 in accordance with specifications of the representative ssSNs selected. Note, the actual ssSNs can be synthetized S32 as part of the above engineering method. Alternatively, the synthesis may be outsourced to an external service provider.

The synthetized sequences are preferably wrapped around the SWCNTs using an exchange protocol. Preferably, use is made of a high-throughput exchange method. For example, the exchange protocol of Lambert et al. can be used to functionalize ssDNA strands on SWCNT surfaces. This method enables the replacement of the surfactant sodium cholate (SC), initially used to suspend the SWCNTs, with ssDNA strands. This allows for high-throughput preparation of ssDNA-SWCNT complexes and facilitates the preparation of low-volume samples, thus reducing the cost of various screenings.

The optical responses of the optical sensors 1 accordingly obtained are subsequently characterized at step S36. Such optical responses are responses to the target molecule of interest. Advantageously, the above exchange method can be applied for high-throughput ssDNA preparation and screening of a large number of ssDNA-SWCNTs for target molecules, either by robotic pipetting or manually.

This way, measurements of the optical responses are obtained, which can then be forwarded S26 to, e.g., the computerized system 100. The latter can subsequently identify S27 one or more top ssSNs among the representative ssSNs. Again, top ssSNs are sequences that lead to highest optical responses of the optical sensors 1. As with the first aspect of the invention, two or more optimization cycles can be performed, with a view to refining the performance of the optical sensors eventually obtained.

Again, the top ssSNs may be refined, e.g., through ML and pattern recognition techniques, as discussed earlier. Eventually, any candidate ssSN may be used to wrap one or more actual ssSNs around SWCNTs, to obtain one or more optimized optical sensor 1.

Referring to FIG. 3B, the optical responses are preferably characterized S36 through near infrared (nIR) fluorescence spectroscopy. The optical response measured for each optical sensor may for instance include a fluorescence peak shift and/or a fluorescence peak intensity change. A peak shift and a peak intensity change can possibly be measured for one or more predetermined excitation wavelengths (preferably two). For example, one may monitor responses of the wrapped SWCNTs based on fluorescence peak shifts and/or intensity changes for low concentrations of glucose.

As noted earlier, one may further rely on nano-sensor arrays. For example, high throughput screening and measurements of different ssDNA sequences and different chiralities of SWCNT can be performed. This approach makes it possible, for example, to screen a nano-biosensor array that includes carbon nanotubes with different chiralities, such as (6,5), (7,5), (7,6), (10,2), and (9,4), each wrapped with different DNA sequences, resulting in distinct optical properties.

In embodiments, a nano-biosensor array is obtained by wrapping a mixture of SWCNTs (with different chiralities) with various single-stranded DNA sequences using a high-throughput exchange method. Each SWCNT chirality thus exhibits a unique spectroscopic signature, which can be represented on a photoluminescence excitation, showing the energy required to excite the different chiralities and the wavelength of the resulting fluorescence. This fluorescent signature can be altered by the presence of a target analyte, modifying the immediate environment of the SWCNTs. These changes in fluorescence can be characterized by shifts in fluorescent intensity, a solvatochromic shift, or a combination of both for a specific chirality. This method was applied to glucose detection; it can be extended to detect other analytes by using a nano-biosensor array with different DNA sequences based on the above methodology, combined with various carbon nanotubes to reveal distinct optical properties specific to a target molecule of interest.

In particular, one may measure optical responses for different chiralities. One possibility is to rely on the (7, 6) and/or (9, 4) chirality peak intensity changes, and/or the (7, 6) and/or (9, 4) chirality peak shifts. In simpler variants, one may focus on a given chirality and solely look at the corresponding peak intensity change. For example, for glucose detection, one may rely on the sole (9, 4) chirality peak intensity change. As the skilled person will understand, other detection schemes can similarly be contemplated.

In that respect, in particularly preferred embodiments of any of the present methods (whether concerning the first or the second aspect of the invention), the target molecule is a molecule of glucose. In that case, the ssSNs are preferably single-stranded sequences of DNA. Beside glucose, however, other target molecules may be contemplated, e.g., NO and OPG biomarkers, as noted earlier. Also, in variants to DNA, use can be made of RNA or XNA, for example.

The present methodology made it possible to predict and test new ssDNA-SWNTs, which can be wrapped around single-walled carbon nanotubes to provide enhanced optical responses to glucose in blood samples. Such nanotubes concern a further aspect of the invention, i.e., a glucose optical sensor comprising a SWCNT wrapped in a single-stranded sequence of DNA (ssDNA). The length of the optimal ssDNA sequences determined is between 10 and 22, as measured in terms of character counts, i.e., the counts of any of the characters A, C, G, and T, in the sequences, corresponding to the four types of bases found in a DNA molecule: adenine (A), cytosine (C), guanine (G), and thymine (T).

In embodiments, the ssDNA comprises the pattern GAC, which appears to correlate well with large fluorescence (9, 4) peak intensity changes. Examples of patterns that provide high-intensity changes are, by order of preference, GACC, GGAC, GACCA, GGACC, GGACCA, or even GGACCAT. The highest intensity change was observed for the GATGGACCAT sequence. Beyond the sole presence of the GAC pattern, better results are usually obtained for sequences of 10 to 22 characters that not only include GAC but, in addition, do not include any of both GCT and TC. The reasons why such patterns lead to higher intensity changes are not yet clear and are currently being investigated.

That said, the presence of the GAC pattern is not systematically required to obtain a large change in intensity. For instance, large intensity changes can be observed for ssDNA containing instead of, or in addition to, GAC one of the following subsequences: ATG, TGG, AAC, GAT, GCC, and GGC.

To summarize, the following sequences have been shown to offer substantial optical responses: GATGAACTAA, AGCGGACCAT, GACGGGCCAT, GTGGGACCTT, CTTACTAGGCCG, GAGGATGGACCATTTA, GGCGGACCAT, GATGGGCCAT, AAGGATGGACCAT, GATGGACCATGATGGACCAT, GAGTGACCAA, GCTTGACCTT, AGAGGACCAT, GACGCCCGCC, GACGGACAAT, CAAGATGGACCATTTT, TAAGATGGACCATTTT, GAGGGACCAT, GGTGGGTCAT, CTTGATGGACCAT, AGGGATGGACCATAAT, GCAGATGGACCAT, GTTGGACCTT, AAAGATGGACCAT, GATGGACCAT, GACGGACTAT, GAAGGACCAT. So, in embodiment, the ssDNA is one of the above sequences.

As noted earlier, it is particularly advantageous to characterize the peak shift and/or intensity change of different chiralities for carbon nanotube wrapped with various single-stranded DNA sequences. This is also true for glucose detection. This approach makes it possible to screen a nano-biosensor array with carbon nanotubes of different chiralities, each wrapped with specific DNA sequences, resulting in distinct optical properties. For instance, a nano-biosensor array can be used to measure a peak shift at the (9, 4) chirality, along with intensity changes at both the (9, 4) and (7, 6) chiralities.

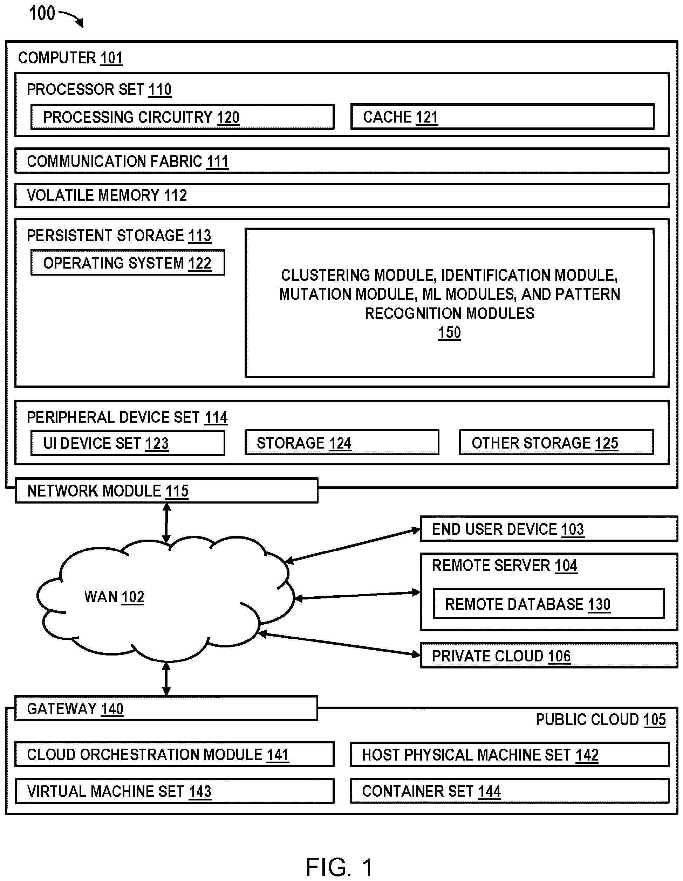

A final aspect concerns a computer program, which comprises software code adapted to perform a method according to any of the embodiments described earlier in reference to the first aspect of the invention, when executed by processing means. The computer program can notably be embodied as a computer program product. The computer program product comprises a computer readable storage medium having program instructions embodied therewith. The program instructions are executable by processing means 110 (e.g., of a computer 101 as shown in FIG. 1) to cause the latter to perform steps of a method according to any of the embodiments described earlier in reference to the first aspect of the invention. Section 3 provides additional implementation details related to computer programs (or computer program products) and computerized systems such as shown in FIG. 1. Interestingly, the process of high-throughput screening and measurement can be assisted by a pipetting robot, as in embodiments. In this case, use can be made of an integrated system where the pipetting robot forms part of the system 100 shown in FIG. 1 (e.g., as a further peripheral device). Further aspects related to such computerized system and computer programs (products) are discussed in detail in section 3.

The above embodiments have been succinctly described in reference to the accompanying drawings and may accommodate a number of variants. Several combinations of the above features may be contemplated. Examples are given in the next sections.

### 2. Specific embodiments

### 2.1. Preferred flow, FIG. 2

The flow shown in FIG. 2 begins with a motif creation step S 10, which leads to a large set of sequences of nucleotides. Such sequences are meant to be used as input to the optimization cycles S20.

During each cycle S20, the sequences of the input set considered are accessed at step S22, in order to be clustered S24. Three distinct clustering schemes are considered, which are based on: folding properties of the sequences, k-mers, and the MAFFT method. An optimal number of clusters is determined S23 for each of the distinct clustering schemes, using the Elbow method. This leads to three sets of clusters, where the sets of clusters overlap in the sequence space. At least one representative sequence is then selected S25 in each cluster from of each clustering scheme, which leads to a pool of representative sequences.

This pool is used as input to a subsequent engineering cycle S30. Each engineering cycle can be regarded as a complementary part of the optimization steps S22 - S25 in a corresponding cycle S20. The representative sequences selected at step S25 are received at step S31, e.g., as a listing, by email. Such sequences are synthetized at step S32, using synthesis techniques that are known *per se.* A high-throughput wrapping techniques is then used S34 to wrap of SWCNTs in the synthetized sequences of nucleotides, leading to N optical sensors, noted Sensor# 1 to Sensor #N in FIG. 2. The N sensors are subsequently screened S35 using a high-throughput robotic setup (or a systematic manual pipetting) to characterize optical responses of the *N* sensors.

The resulting optical response measurements are stored and assessed at step S38. In the event that the results are already satisfactory, the process could stop there. The optical sensors obtained can then be used to detect the target molecule. But, without being fully satisfactory yet, these results could already prove sufficient for additional optimization by ML and pattern recognition. In this case, the process would resume at step S42, see below. However, the most likely scenario is that the obtained sequences need to be further optimized, at least after the first series of steps S22 - S25 and the first engineering cycle S30. In this case, the first optimization cycle resumes at step S27. Doing so is preferable anyway, if only to further optimize, if only locally, the sequences obtained after the first series of steps S22 - S25.

The measurements are accessed at step S26. From these measurements, one or more top sequences are identified at step S27. If the current optimization cycle is meant to be the last (S28: Yes), the process can either stop here (not shown) or resume at S40. Else (S28: No), each of the top sequences is mutated S29 several times to obtain a superset of the sequences, including the top sequence and its mutants. This superset is then used as a new input set to start a new optimization cycle S20.

The reference S40 denotes a further optimization process, based on ML and pattern recognition techniques. At step S42, an odd number (larger than or equal to three) of ML classifiers are trained on all sequences for which measurements are available so far, with a view to performing S44 classifications for each sequence of test set. The classifications are followed by a majority voting S46. The sequences that lead to favourable response classification are then subject to pattern recognition S48 to identify those patterns that perform best and/or generate one or more new sequences. If necessary, the process loops back to step S44, to perform new classifications.

Eventually, the best sequence (as inferred, or predicted) are selected S49 for subsequent characterization. That is, the process resumes at step S32, whereby the selected sequences are synthetized for subsequent optical characterization.

### 2.2. Particularly preferred embodiments (FIGS. 3A - 6B)

FIGS. 3A - 6B illustrate a process integrating optical sensing and machine learning for detecting glucose using ssDNA-SWCNT sensors.

In detail, FIGS. 4A - 5C illustrates an exploration scheme approach using both unsupervised and supervised ML techniques. The idea is to explore a space (FIG. 4A) of DNA sequences by clustering (FIG. 4B) such sequences. Two million DNA sequences with different base and length combinations are clustered using different methods. FIG. 6A illustrates the clustering strategy, which makes use of three different clustering methods (MAFFT, k-mer, and folding properties) for navigating the large input space of DNA sequences. FIG. 6B illustrates the use of the Elbow method for obtaining an optimal number of clusters. The number of clusters obtained is fairly small when using the MAFFT method. This number increases when using folding properties. The k-mer clustering method is based on a specific k-mer (k = 3); The sequences are then clustered based on the k-mer frequency across the various sequences.

Representative sequences are then selected in each cluster (FIG. 4B) to obtain a diverse sampling of the DNA sequences. Next, optical sensors are fabricated (FIG. 5B) by wrapping SWCNTs in DNA sequences synthetized in accordance with the representative sequences selected.

The optical response of each optical sensor is subsequently measured. FIG. 3A illustrates the optical response measurement of an ssDNA-SWCNT optical sensor 1 to a target molecule (glucose). Two quantities are observed in this example, which are the peak shift and intensity change (FIG. 3B) using nIR fluorescence at a specific excitation wavelength. Alternatively, one may measure the fluorescence response of an optical sensor array (not shown), including nanotubes with various SWCNT chiralities and DNA sequences, to the target molecule (e.g., glucose). That is, one may screen the fluorescence response to glucose of ssDNA-SWCNT optical sensor arrays, whereby the emitted fluorescence, which may undergo a peak shift and/or intensity change in presence of the analyte, can be measured through a photoluminescence map based on the response of different SWCNT chiralities.

These measurements obtained are then exploited to identify the top sequence, i.e., the sequence that led to the highest optical response, among the representative sequences selected. The top sequence (i.e., the most responsive sequence) is selected for three mutations in bases and lengths (FIG. 4C). Next, a superset of the top sequence, including its mutants, is formed, with a view to starting another optimization cycle based on this superset. The superset still forms a large library. So, in order to find a local optimum, the clustering method is performed again to identify new representative sequences. The process stops after the second optimization cycle.

An enhancement process is subsequently started, which can also be regarded as a further optimization. FIG. 5A (from left to right): Three ML classifiers are trained on all DNA sequences, for which measurements are available. The trained classifiers are then used to predict the behaviour of a large set of ssDNA-SWCNTs (i.e., SWCNTs wrapped in a large set of sequences) for glucose detection. Pattern recognition techniques (not shown) are used to identify the best k-mers and analyse the positions of DNA spots that best interact with glucose. In the final step, the best DNA sequence is selected to further investigate the interaction between glucose and the ssDNA-SWCNT sensor.

The above approach makes it possible to identify or predict optical responses for a large space of input sequences (FIG. 5C). As an example, FIG. 6C illustrates the experimental (9, 4) intensity change in response to glucose detection at 7.5 mM by ssDNA-SWCNT, using a set of diverse ssDNA sequences as obtained after a first clustering step. Similar graphs were obtained for the (7, 6) intensity change and the (9, 4) peak shift (not shown). During subsequent optimization steps, additional sequences were identified, which turned out to be even more responsive to glucose. Note, as seen in FIG. 6C, sequences obtained using a clustering method based on MAFFT, folding properties, or k-mers, are substantially more responsive than random sequences and sequences obtained through the so-called Rational method, using different lengths (X) of DNA sequences such as (GT)x, (AC)x, and (AT)x.

The chiralities of the nanotubes are specified by the chiral vectors (*n, m*)*,* which define the roll-up angle, length, and diameter. The pairs of integers (*n, m*) determine whether the SWCNTs are metallic (*n = m*)*,* semi-metallic (|*n - m*| = 3 *k*, where *k* is an integer), or semi-conducting (In *- m*| = 3 *k* ± 1). In preferred embodiments as disclosed herein, use is made of so-called HiPCO single-walled carbon nanotubes, which are semi-conducting SWCNTs with different chiralities. Such SWCNTs are subjected to energetic incident light (500 - 900 nm), resulting in the formation of an exciton, which can move along the conducting nanotubes. This exciton relaxes in the energetic sub-bands of the conduction band and then radially relaxes toward the valence band (900 - 1400 nm), generating the characteristic near-infrared fluorescence of the SWCNTs. For completeness, in the present context, the SWCNTs typically have diameters between 0.5 and 2.0 nanometres, and preferably between 0.7 and 1.0 nanometres. Their lengths can for example be of between 100 and 1000 nanometres.

### 2.3 Examples of application

### 2.3.1. Improved ssDNA-SWCNTs sensor for nitric oxide detection for inflammation monitoring

The present inventors developed a guided approach to engineer optical ssDNA-SWCNT sensors. This approach is based on optimization cycles as described earlier, containing clustering steps, as well as ML techniques based on NNs (CNN model learning) and pattern recognition. Using a combination of clustering and learning, they designed an optical sensor 1 for nitric oxide (NO), a pro-inflammatory mediator that induces inflammation. They demonstrated a 10-fold enhancement in sensor response compared with state-of-the-art sensors based on the AT15 sequence. Accordingly, their approach manifestly provides a powerful means of overcoming existing bottlenecks in SWCNT sensor design, ushering in a new generation of near-infrared technologies for biomedical research and clinical diagnostics.

### 2.3.2. Application to OPG protein nanosensor engineering

The present inventors further developed a novel approach for engineering OPG protein nanosensors by using unsupervised and supervised ML. The methodology leverages both unsupervised clustering and supervised learning techniques to optimize ssDNA-SWCNT single-stranded DNA-SWCNT complexes for improved sensor performance. This approach is based on optimization cycles using clustering techniques as described earlier, and subsequent CNN model learning. The process begins by clustering a large library of 10⁶ ssDNA sequences, from which representative sequences are selected for nanosensor preparation and photoluminescence (PL) screening. Sequences exhibiting enhanced features, such as improved selectivity and sensitivity, are selected and subjected to iterative cycles of mutation and further clustering. The data from these cycles is used to train a supervised ML model, which, after validation, predicts high-performing ssDNA-SWCNT complexes for future rounds of optimization. By systematically exploring the sequence space, this MI,-assisted process accelerates the discovery of highly selective and sensitive nanosensors, enabling rapid advancement in the development of OPG protein detection technologies.

### 3. Technical implementation details

Various aspects of the present disclosure are described by narrative text, flowcharts, block diagrams of computer systems and/or block diagrams of the machine logic included in computer program embodiments or computer program product (CPP) embodiments. With respect to any flowcharts, depending upon the technology involved, the operations can be performed in a different order than what is shown in a given flowchart. For example, again depending upon the technology involved, two operations shown in successive flowchart blocks may be performed in reverse order, as a single integrated step, concurrently, or in a manner at least partially overlapping in time.

CPP embodiment is a term used in the present disclosure to describe any set of one, or more, storage media (also called mediums) collectively included in a set of one, or more, storage devices that collectively include machine readable code corresponding to instructions and/or data for performing computer operations specified in a claim. A storage device is any tangible device that can retain and store instructions for use by a computer processor. Without limitation, the computer readable storage medium may be an electronic storage medium, a magnetic storage medium, an optical storage medium, an electromagnetic storage medium, a semiconductor storage medium, a mechanical storage medium, or any suitable combination of the foregoing. Some known types of storage devices that include these mediums include: hard disk, random access memory (RAM), read-only memory (ROM), erasable programmable read-only memory (EPROM or Flash memory), static random-access memory (SRAM), compact disc read-only memory (CD-ROM), digital versatile disk (DVD), memory stick, floppy disk, mechanically encoded device (such as punch cards or pits / lands formed in a major surface of a disc) or any suitable combination of the foregoing.

A computer readable storage medium is not to be construed as storage in the form of transitory signals per se, such as radio waves or other freely propagating electromagnetic waves, electromagnetic waves propagating through a waveguide, light pulses passing through a fibre optic cable, electrical signals communicated through a wire, and/or other transmission media. As will be understood by the skilled person, data is typically moved, on occasions, during normal operations of a storage device, such as during access, de-fragmentation, or garbage collection, but this does not render the storage device as transitory because the data is not transitory while it is stored.

Computing environment 100 is an example of an environment for the execution of at least some of the computer code involved in performing the present methods, such as steps of clustering, identifications, mutations, ML training and inferences, and pattern recognition (see block 150) as described herein. In addition to block 150, the computing environment 100 includes, for example, computer 101, wide area network (WAN) 102, end user device (EUD) 103, remote server 104, public cloud 105, and private cloud 106. In this embodiment, computer 101 includes processor set 110 (including processing circuitry 120 and cache 121), communication fabric 111, volatile memory 112, persistent storage 113 (including operating system 122 and block 150, as identified above), peripheral device set 114 (including user interface (UI) device set 123, storage 124, and peripheral storage 125), and network module 115. Remote server 104 includes remote database 130. Public cloud 105 includes gateway 140, cloud orchestration module 141, host physical machine set 142, virtual machine set 143, and container set 144.

COMPUTER 101 may take the form of a desktop computer, laptop computer, tablet computer, smart phone, smart watch or other wearable computer, mainframe computer, or any other form of computer or mobile device now known or to be developed in the future that is capable of running a program, accessing a network, or querying a database, such as remote database 130. Computer 101 is assumed to include one or several interface units that allows it to receive 3D images. As is well understood in the art of computer technology, and depending upon the technology, performance of a computer-implemented method may be distributed among multiple computers and/or between multiple locations. That said, the following discussion is focused on a single computer, specifically computer 101, in the interest of simplicity. Computer 101 may possibly be in a cloud.

PROCESSOR SET 110 includes one, or more, computer processors of any type now known or to be developed in the future. Processing circuitry 120 may be distributed over multiple packages, for example, multiple, coordinated integrated circuit chips. Processing circuitry 120 may implement multiple processor threads and/or multiple processor cores. Cache 121 is memory that is located in the processor chip package(s) and is typically used for data or code that should be available for rapid access by the threads or cores running on processor set 110. Cache memories are typically organized into multiple levels depending upon relative proximity to the processing circuitry. Alternatively, some, or all, of the cache for the processor set may be located off chip.

Computer readable program instructions are typically loaded onto computer 101 to cause a series of operational steps to be performed by processor set 110 of computer 101 and thereby effect a computer-implemented method, such that the instructions thus executed will instantiate the methods specified in flowcharts and/or narrative descriptions of computer-implemented methods included in this document (collectively referred to as the inventive methods). These computer readable program instructions are stored in various types of computer readable storage media, such as cache 121 and the other storage media discussed below. The program instructions, and associated data, are accessed by processor set 110 to control and direct performance of the inventive methods. In computing environment 100, at least some of the instructions for performing the inventive methods may be stored in block 150 in persistent storage 113.

COMMUNICATION FABRIC 111 is the signal conduction path that allows the various components of computer 101 to communicate with each other. Typically, this fabric is made of switches and electrically conductive paths, such as the switches and electrically conductive paths that make up buses, bridges, physical input / output ports and the like. Other types of signal communication paths may be used, such as fibre optic communication paths and/or wireless communication paths.

VOLATILE MEMORY 112 is any type of volatile memory now known or to be developed in the future. Examples include dynamic type random access memory (RAM) or static type RAM. Typically, volatile memory 112 is characterized by random access, but this is not required. In computer 101, the volatile memory 112 is located in a single package and is internal to computer 101, but, alternatively or additionally, the volatile memory may be distributed over multiple packages and/or located externally with respect to computer 101.

PERSISTENT STORAGE 113 is any form of non-volatile storage for computers that is now known or to be developed in the future. The non-volatility of this storage means that the stored data is maintained regardless of whether power is being supplied to computer 101 and/or directly to persistent storage 113. Persistent storage 113 may be a read only memory (ROM), but typically at least a portion of the persistent storage allows writing of data, deletion of data and re-writing of data. Some familiar forms of persistent storage include magnetic disks and solid-state storage devices. Operating system 122 may take several forms, such as various known proprietary operating systems or open-source Portable Operating System Interface-type operating systems that employ a kernel. The code included in block 150 typically includes at least some of the computer code involved in performing the inventive methods.

PERIPHERAL DEVICE SET 114 includes the set of peripheral devices of computer 101. Data communication connections between the peripheral devices and the other components of computer 101 may be implemented in various ways, such as Bluetooth connections, Near-Field Communication (NFC) connections, connections made by cables (such as universal serial bus (USB) type cables), insertion-type connections (for example, secure digital (SD) card), connections made through local area communication networks and even connections made through wide area networks such as the internet. In various embodiments, UI device set 123 may include components such as a display screen, speaker, microphone, wearable devices (such as goggles and smart watches), keyboard, mouse, printer, touchpad, game controllers, and haptic devices. Storage 124 is external storage, such as an external hard drive, or insertable storage, such as an SD card. Storage 124 may be persistent and/or volatile. In embodiments where computer 101 is required to have a large amount of storage (for example, where computer 101 locally stores and manages a large set of 3D images) then this storage may be provided by peripheral storage devices 125 designed for storing very large amounts of data, such as a storage area network (SAN) that is shared by multiple, geographically distributed computers.

NETWORK MODULE 115 is a collection of computer software, hardware, and firmware, which allows computer 101 to communicate with other computers through WAN 102. Network module 115 may include hardware, such as modems or Wi-Fi signal transceivers, software for packetizing and/or de-packetizing data for communication network transmission, and/or web browser software for communicating data over the internet. In some embodiments, network control functions and network forwarding functions of network module 115 are performed on the same physical hardware device. In other embodiments (for example, embodiments that utilize software-defined networking (SDN)), the control functions and the forwarding functions of network module 115 are performed on physically separate devices, such that the control functions manage several different network hardware devices. Computer readable program instructions for performing the inventive methods can typically be downloaded to computer 101 from an external computer or external storage device through a network adapter card or network interface included in network module 115.

WAN 102 is any wide area network (for example, the internet) capable of communicating computer data over non-local distances by any technology for communicating computer data, now known or to be developed in the future. In some embodiments, the WAN 102 may be replaced and/or supplemented by local area networks (LANs) designed to communicate data between devices located in a local area, such as a Wi-Fi network. The WAN and/or LANs typically include computer hardware such as copper transmission cables, optical transmission fibres, wireless transmission, routers, firewalls, switches, gateway computers and edge servers.

END USER DEVICE (EUD) 103 is any computer system that is used and controlled by an end user (for example, a customer of an enterprise that operates computer 101) and may take any of the forms discussed above in connection with computer 101. EUD 103 typically receives helpful and useful data from the operations of computer 101. For example, in a hypothetical case where computer 101 is designed to provide a recommendation to an end user, this recommendation would typically be communicated from network module 115 of computer 101 through WAN 102 to EUD 103. In this way, EUD 103 can display, or otherwise present, the recommendation to an end user. In some embodiments, EUD 103 may be a client device, such as thin client, heavy client, mainframe computer, desktop computer and so on.

REMOTE SERVER 104 is any computer system that serves at least some data and/or functionality to computer 101. Remote server 104 may be controlled and used by the same entity that operates computer 101. Remote server 104 represents the machine(s) that collect and store helpful and useful data for use by other computers, such as computer 101. For example, in a hypothetical case where computer 101 is designed and programmed to provide a recommendation based on historical data, then this historical data may be provided to computer 101 from remote database 130 of remote server 104.

PUBLIC CLOUD 105 is any computer system available for use by multiple entities that provides on-demand availability of computer system resources and/or other computer capabilities, especially data storage (cloud storage) and computing power, without direct active management by the user. Cloud computing typically leverages sharing of resources to achieve coherence and economies of scale. The direct and active management of the computing resources of public cloud 105 is performed by the computer hardware and/or software of cloud orchestration module 141. The computing resources provided by public cloud 105 are typically implemented by virtual computing environments that run on various computers making up the computers of host physical machine set 142, which is the universe of physical computers in and/or available to public cloud 105. The virtual computing environments (VCEs) typically take the form of virtual machines from virtual machine set 143 and/or containers from container set 144. It is understood that these VCEs may be stored as images and may be transferred among and between the various physical machine hosts, either as images or after instantiation of the VCE. Cloud orchestration module 141 manages the transfer and storage of images, deploys new instantiations of VCEs and manages active instantiations of VCE deployments. Gateway 140 is the collection of computer software, hardware, and firmware that allows public cloud 105 to communicate through WAN 102.

Some further explanation of virtualized computing environments (VCEs) will now be provided. VCEs can be stored as images. A new active instance of the VCE can be instantiated from the image. Two familiar types of VCEs are virtual machines and containers. A container is a VCE that uses operating-system-level virtualization. This refers to an operating system feature in which the kernel allows the existence of multiple isolated user-space instances, called containers. These isolated user-space instances typically behave as real computers from the point of view of programs running in them. A computer program running on an ordinary operating system can utilize all resources of that computer, such as connected devices, files and folders, network shares, CPU power, and quantifiable hardware capabilities. However, programs running inside a container can only use the contents of the container and devices assigned to the container, a feature which is known as containerization.

PRIVATE CLOUD 106 is similar to public cloud 105, except that the computing resources are only available for use by a single enterprise or organization. While private cloud 106 is depicted as being in communication with WAN 102, in other embodiments a private cloud may be disconnected from the internet entirely and only accessible through a local/private network. A hybrid cloud is a composition of multiple clouds of different types (for example, private, community or public cloud types), often respectively implemented by different vendors. Each of the multiple clouds remains a separate and discrete entity, but the larger hybrid cloud architecture is bound together by standardized or proprietary technology that enables orchestration, management, and/or data/application portability between the multiple constituent clouds. In this embodiment, public cloud 105 and private cloud 106 are both part of a larger hybrid cloud.

While the present invention has been described with reference to a limited number of embodiments, variants, and the accompanying drawings, it will be understood by those skilled in the art that various changes may be made, and equivalents may be substituted without departing from the scope of the present invention. In particular, a feature (device-like or method-like) recited in a given embodiment, variant or shown in a drawing may be combined with or replace another feature in another embodiment, variant or drawing, without departing from the scope of the present invention. Various combinations of the features described in respect of any of the above embodiments or variants may accordingly be contemplated, that remain within the scope of the appended claims. In addition, many minor modifications may be made to adapt a particular situation or material to the teachings of the present invention without departing from its scope. Therefore, it is intended that the present invention is not limited to the particular embodiments disclosed, but that the present invention will include all embodiments falling within the scope of the appended claims. In addition, many other variants than explicitly touched above can be contemplated. For example, other NN architectures, clustering techniques, and pattern recognition techniques can be contemplated.

## Claims

1. A computer-implemented method of optimizing an optical sensor (1) comprising a single-walled carbon nanotube (2), or SWCNT, wrapped in single-stranded sequence (3) of nucleotides, or ssSN, wherein the method comprises
performing (S20) two or more optimization cycles, wherein each cycle of the optimization cycles includes:
clustering (S24) ssSNs of an input set of ssSNs to obtain clusters (C11 - C33) and selecting (S25) representative ssSNs of the clusters obtained;
accessing (S26) measurements of optical responses of optical sensors (1) to a target molecule, the optical sensors including SWCNTs wrapped in actual ssSNs, the latter synthetized in accordance with the representative ssSNs selected; and
identifying (S27), among the representative ssSNs, a top ssSN that led to a highest one of the optical responses, and
mutating (S29) the top ssSN identified at each of the optimization cycles but a last cycle thereof to obtain mutants and form a superset of sequences including the top ssSN and said mutants, prior to performing (S20) a next one of the optimization cycles, using this superset as a new input set of ssSNs.

2. The method according to claim **1,** wherein, at mutating (S29) the top ssSN,
the top ssSN is mutated (S29) several times through changes in both base and length.

3. The method according to claim **2,** wherein, at mutating (S29) the top ssSN,
the top ssSN is mutated (S29) by introducing *n_{bsm}* base sequence mutations and adding *n_{ba}* bases at extremities of the top ssSN, where each of *n_{bsm}* and *n_{ba}* is equal to 1, 2, or 3.

4. The method according to any one of claims **1** to **3,** wherein, at said each cycle,
the ssSNs are clustered (S24) according to one or more distance functions based on *N_{d}* distinct sequence properties of the ssSNs, whereby the clusters (C11 - C33) obtained at said each cycle include at least *N_{d}* parallel sets (S1 - S3) of clusters, where *N_{d} ≥* 2, preferably *N_{d} =* 3, and
the representative ssSNs are selected (S25) in each cluster (C11 - C33) of the distinct sets (S1 - S3) of clusters obtained, wherein each of the representative ssSNs is preferably selected (S25) based on its distance to a centre of a respective one of the clusters (C11 - C33).

5. The method according to claim **4,** wherein the distinct sequence properties include two or more, preferably each, of:
an alignment similarity of the sequences,
a k-mer frequency in the sequences, where k is preferably of between 3 and 7, and more preferably equal to 3, and
folding properties of the sequences.

6. The method according to claim **1** or **5,** wherein said each cycle further comprises,
determining (S23) optimal numbers of clusters for respective ones of the *N_{d}* parallel sets (S1 - S3), preferably using the Elbow method, whereby the ssSNs are clustered (S24) in accordance with the optimal numbers of clusters determined, preferably using an unsupervised machine learning clustering method, more preferably the *κ*-means clustering method.

7. The method according to any one of claims **1** to **6,** wherein the method further comprises, after said last cycle,
determining (S40)) one or more candidate ssSNs that potentially provide highest optical responses to the target molecule, based on at least a subset, preferably a full set of, all representative ssSNs corresponding to all optical sensors (1) for which measurements of optical responses were accessed throughout the optimization cycles, using (S44, S48) inference techniques relying on one or more computational models.

8. The method according to claim 7, wherein
said one or more models involve one or more machine learning models, and
the one or more candidate ssSNs are determined (S40) by:
training (S42) each model of the one or more machine learning models on a training dataset consisting of pairs of inputs and outputs, where the inputs correspond to at least a subset of said all representative ssSNs, respectively, and the outputs are based on measurements of optical responses as accessed (S26) for respective ones of the optical sensors (1); and
running (S44) said each model, once trained, on a test set of ssSNs to infer (S44) respective optical response performances, whereby the one or more candidate ssSNs are eventually determined (S48) based on the optical response performances inferred.

9. The method according to claim **8,** wherein
the set of one or more machine learning models comprises an odd number of at least three machine learning models, each configured as a classifier, whereby the optical response performances are inferred (S44) as classifications into classes that include, preferably consist of, a first class corresponding to a high optical response and a second class corresponding to a low optical response, and
the method further comprises performing (S46) a majority vote based on the classifications obtained from each model of the set of machine learning models.

10. The method according to claim **8** or **9,** wherein the method further comprises
performing (S48) pattern recognition on test sequences of the test set of ssSNs based on the optical response performances inferred therefor to identify sequence patterns that respond best, optically, to the target molecule, whereby the one or more candidate ssSNs are eventually determined based on the sequence patterns identified, and
preferably predicting one or more new sequences based on the identified patterns, whereby the one or more candidate ssSNs are eventually determined based on the one or more new sequences predicted.

11. The method according to claim **10,** wherein said sequence patterns include patterns in terms of one or more, preferably two, and more preferably three, of k-mers, base positions, and lengths, of the test sequences.

12. A method of engineering one or more optical sensors (1), each comprising a single-walled carbon nanotube (SWCNT) wrapped in a single-stranded sequence of nucleotides (ssSN), wherein the method comprises performing two or more optimization cycles, each including:
receiving (S31), from a computerized system, representative ssSNs as selected during a corresponding optimization cycle of the method according to any one of claims **1** to **11;**
wrapping (S34) actual ssSNs around SWCNTs to obtain optical sensors (1), the actual ssSNs synthetized (S32) in accordance with the representative ssSNs selected, wherein the actual ssSNs are preferably wrapped around the SWCNTs using an exchange protocol; and
characterizing (S36) optical responses of the optical sensors (1) to a target molecule to obtain measurements of said optical responses; and
forwarding (S26) measurements of the optical responses to the computerized system for it to identify (S27), among the representative ssSNs, one or more top ssSNs that led to highest optical responses of the optical sensors (1).

13. The method according to claim **12,** wherein said optical responses
are characterized (S36) through near infrared fluorescence spectroscopy, and
include, for each of the optical sensors (1), a fluorescence peak shift and/or a fluorescence peak intensity change, each obtained for one or more predetermined excitation wavelengths.

14. The method according to any one of claims **1** to **13,** wherein
the target molecule is glucose and the ssSNs are single-stranded sequences of DNA.

15. A computer program comprising software code adapted to perform a method of optimizing an optical sensor (1) according to any one of claims **1** to **11** when executed by processing means.
